# EUROPEAN PATENT APPLICATION

(11) **EP 3 061 823 A1**
(43) Date of publication of application: **31.08.2016**
(21) Application number: 15156548.8
(22) Date of filing: 25.02.2015
(51) Int. Cl.: C12N 15/10

(54) **Method for extracting nucleic acids from an agarose matrix**

(71) Applicant: QIAGEN GmbH, 40724 Hilden (DE)
(72) Inventor: Hucklenbroich, Jörg, 42929 Wermelskirchen (DE); Singer, Thorsten, 42699 Solingen (DE)
(74) Representative: f & e patent

(57) **Abstract**

The present invention provides a method for extracting one or more nucleic acids from a sample comprising gelled agarose (an agarose matrix) as well as kit for carrying out this method.

## Description

The present invention relates to a method for extracting one or more nucleic acids from a sample comprising gelled agarose (agarose matrix) and at least one nucleic acid, preferably from agarose gels, by using a solid phase, which selectively binds nucleic acids in the presence of a binding mediating agent without the need for chaotropic substances, as well as to a kit for carrying out said method.

For the separation of nucleic acids, including ribonucleic acids (RNA) and desoxyribonucleic acids (DNA), on the basis of their size, fractionated agarose gel electrophoresis is the method of choice. Nucleic acids isolated from agarose gels are frequently used as templates for polymerase chain reaction (PCR) or reverse transcript-PCR (RT-PCR) as well as for hybridization, sequencing, ligation, and various other recombinant molecular techniques.

All these downstream analyses depend upon the quality and purity of the nucleic acids isolated from the agarose matrix, i.e. the method employed for extracting the nucleic acids from the matrix. In attempt to recover nucleic acids, mainly DNA, from agarose gels in an intact form and free of contaminants, several methods have been developed during the past 30 years. These methods include for example processes for physical gel destruction, like the so-called "freeze and squeeze" procedure (R.W.J. Thuring, J.N.P. Anders, P. Borst Anal. Biochem. 1975, 66, 213*)* or the so-called "crush and soak" method.

Physical extrusion of DNA molecules from a gel (or a part thereof) includes e.g. DNA extraction by electro-elution inside a dialysis bag into an electrophoresis buffer, from which the DNA can be recovered by precipitation. According to another procedure, a small trough is cut in the agarose gel just in front of the DNA band to be recovered. The DNA then is electrophoretically eluted onto a diethylaminoethyl (DEAE)-cellulose paper strip, an affinity membrane, a gel insert comprising a mixture of sodium acetate and sucrose, or a dialysis tubing (P.N. Hengen Trends in Biochemical Sciences 1994, 19 (9), 388).

The gel may also be dissolved chemically or digested enzymatically to release the nucleic acids into solution. For example, low melting temperature (LMT) agarose can be digested using GELase (Epicentre Biotechnologies, Madison, WI, USA), a DNase-free combination of enzymes comprising beta-agarase, which digests the agarose into multimeric subunits. However, digestion takes several hours to overnight and in general additional steps of phenol extraction and/or precipitation are required for purifying the nucleic acid.

Following the fundamental procedure described by Vogelstein and Gillespie (Proc. Nat. Acad. Sci. USA 1979, 76 (2), 615) several methods have been developed, wherein at first the agarose gel matrix is thermally and/or chemically dissolved in the presence of a chaotropic salt such as guanidine hydrochloride, guanidinium thiocyanate, guanidinium isothiocyanate, or sodium iodide. The nucleic acids are then selectively bound to silica particles, so-called "glass milk", diatomaceous earth, or further commercially available binding matrices/resins in the presence of a high concentration (usually > 2 M) of one or more of the aforementioned chaotropic substances. Based on this general principle, various kits for DNA extraction from agarose gels are commercially available, for example the GENECLEAN kit from Bio101 (Davidson, MC, USA), the agarose gel extraction kit from Jena Bioscience (Jena, Germany) or the QIAquick and QIAex kits, both from Qiagen (Hilden, Germany).

One drawback of these chaotrope-mediated methods is the fact that the used chaotropic salts are toxic and/or corrosive and moreover usually are required in a concentration of more than 2 M in the binding solution. Furthermore, using these methods, recovery of nucleic acid fragments having a length of less than 150 base pairs (bp) often is poor.

Accordingly, the object of the present invention was to provide a method for extracting one or more nucleic acids from a sample comprising gelled agarose (an agarose matrix) which avoids the use of the chaotropic substances, in particular of guanidine hydrochloride, guanidinium (iso)thiocyanate, or sodium iodide.

This object is met by the method of the present invention. The present invention provides a method for extracting one or more nucleic acids from a sample comprising gelled agarose (agarose matrix) and at least one nucleic acid, preferably from agarose gels of nucleic acids, comprising the steps of: at least partly liquefying the agarose matrix and separating the nucleic acid from the sample using a solid phase, which selectively binds the nucleic acid in presence of a binding mediating agent, wherein neither the binding mediating agent nor any other compound added to the sample during the procedure comprises a chaotropic substance, selected from the group comprising sodium perchlorate, sodium iodide, potassium iodide, guanidinium (iso)thiocyanate, and guanidine hydrochloride. Preferably neither the binding mediating agent nor any other compound added to the sample during the whole procedure nor the sample itself comprise more than 0.5 M of any chaotropic substance. Preferably any of the added components comprises less than 0.1 M, more preferably less than 0.05 M of any chaotropic substance.

It has surprisingly been found, that a nucleic acid can be extracted from agarose matrices, in particular excised agarose gel bands of DNA or RNA electrophoresis gels by an extraction procedure which preferably avoids the use of any chaotopic substances, but nevertheless makes use of the solid phase-based separating step typically employed in these chaotrope-based methods. Thus, the method of the present invention combines the known advantages of solid phase-based separation technology and the further advantage that no toxic and/or corrosive chaotropic substances have to be employed during the whole extracting procedure. This is possible, since it has surprisingly been found that a non-chaotropic binding mediating agent is able to selectively mediate the binding between the solid phase and the nucleic acids, even in the presence of residual agarose and/or substances used to dissolve and/or remove the agarose matrix, for example precipitating agents used for removing the agarose matrix.

By using the method of the present invention nucleic acids can be extracted from any sample comprising gelled agarose / an agarose matrix. Preferably agarose gels from gel electrophoresis (or parts thereof) are used as a sample in the present invention. Preferably, parts of an agarose gel, i.e. parts including particular bands of interest excised from an agarose gel using for example a razor blade or a scalpel may be used. In the following the term "agarose gel" comprises both, a complete gel as well as excised parts of such a gel, e.g. including nucleic acid fragments.

Agarose is a commercial available linear polymer, from which agarose gels are prepared by melting a defined amount of said polymer in an appropriate buffer until a clear solution is obtained. Upon cooling, the melted agarose forms a block gel comprising a continuous filamentous matrix forming rather large pores, the diameter of which depends upon the concentration of the agarose. Using the method of the present invention, nucleic acids can be extracted from agarose gels of any agarose concentration commonly used for gel electrophoresis. Said concentration is not limiting the present invention and preferably may be in the range of from 0.2 to 5 %, preferably of from 0.3 to 3 %, more preferably of from 0.5 to 1.5 %, and most preferably of about 0.8 % (w/v) agarose, based on the amount of commercially available agarose suspended in the buffer before melting the agarose to form the gel.

The method of the present invention further allows the extraction of nucleic acids from gels of any buffer commonly used in gel electrophoresis, including for example Tris/acetate/EDTA (TAE), Tris/borate/EDTA (TBE), and lithium borate (LB) buffer for DNA agarose electrophoresis and formaldehyde buffers for RNA electrophoresis, respectively.

In a first step, the agarose matrix of the sample is at least partly liquefied. In terms of the present invention the term "at least partly liquefying" comprises any step or range of steps for transforming the agarose matrix from a rather dimensionally stable, non-flowing state, i.e. the gel, into a fluid (flowing) state. This can be achieved by any steps or range of steps known from the state of the art for this purpose, e.g. by dissolving the matrix in an appropriate agarose gel lysis buffer, under the *proviso* that this lysis buffer does not contain any of the chaotropic substances mentioned above. Preferably, said agarose gel lysis buffer does not contain more than 0.5 M, more preferably not more than 0.1 M, even more preferably not more than 0.05 M of any chaotropic substance. Most preferably, the agarose gel lysis buffer does not contain any chaotropic substance at all. Alternatively and/or additionally, the agarose matrix may also be liquefied by melting, as described below.

In terms of the present invention a binding mediating agent is a non-chaotropic substance which mediates selective binding between the solid phase and the nucleic acids. By "mediating selective binding" it is meant, that in the absence of the binding mediating agent the nucleic acids do not bind to the solid phase or at least only in a limited extent (e.g. less than 15 %, preferably less than 10 %, more preferably less than 5%, even more preferably less than 2 % and most preferably less than 0.5 %, based on the total amount of nucleic acids bound to the solid phase in the presence of the optimum amount of binding agent), while in the presence of said binding mediating agent the nucleic acids present in a sample can be attached to the solid phase, for example via ionic or electrostatic interactions, without being limited to these. Preferably all kind of nucleic acids present in the sample may be bound to the solid phase in the presence of said binding mediating agent or at least all ribonucleic acids and/or desoxyribonucleic acids present in the sample, irrespective of their particular substitution pattern. Thus, using the method of the present invention it is not necessary to introduce an particular catch-and-release substitution pattern in the nucleic acid to be isolated or to specifically adapt the solid phase in such a way. Other compounds present in the sample, such as for example residual agarose, salts, etc. (except for the binding mediating agent) are not bound to the solid phase in a relevant amount (i. e. less than 2 %, preferably less than 1 %, more preferably less than 0.5 %, based on the total amount of nucleic acids bound to the solid phase in the presence of the optimum amount of binding agent).

Chaotropic substances denature proteins, increase the solubility of non-polar substances in water and destroy hydrophobic interactions. In addition, they also mediate the binding of the nucleic acids to selected solid phases. The Hofmeister series (also called lyotropic series) ranks the relative influence of ions on the physical behavior of water and aqueous processes, such as colloidal assembly or protein folding:
CO₃²⁻ > SO₄²⁻ > S₂O₃²⁻ > H₂PO₄⁻ > F⁻ > Cl⁻ > Br⁻> NO₃⁻ > I⁻ > ClO₄⁻ > SCN⁻ [for anions] and
NH₄⁺ > K⁺ > Na⁺ > Li⁺ > Mg²⁺ > Ca²⁺ > guanidinium [for cations].

"Early" members of the series, i.e. members on the left, increase the surface tension of the solvent and decrease the solubility of non-polar molecules, such as proteins (so-called "salting out"/aggregation), while the "later" members (on the right) decrease the solvent's surface tension and increase the solubility of non-polar molecules (so-called "salting in"/solubilizing). The specific order of two ions in the series may vary depending upon the scientific reference, in particular for ions positioned in the middle of the series, it is however generally accepted that iodide, perchlorate, (iso)thiocyanate, and/or guanidinium ions are chaotropic ions, while sulphate and ammonium ions, for example, are non-chaotropic ions. Thus, in terms of the present invention a chaotrope in particular is a water-soluble salt comprising iodide, perchlorate, (iso)thiocyanate, and/or guanidinium ions, such as for example sodium perchlorate, sodium iodide, potassium iodide, guanidinium (iso)thiocyanate, and guanidine hydrochloride.

In the method of the present invention these chaotropic substances are required neither for liquefying the agarose matrix nor for selectively binding the nucleic acid to the solid phase in order to essentially separate the nucleic acids from the sample. Thus, preferably neither the binding mediating agent nor any other compound or component added to the sample during the procedure comprises a significant amount of a chaotropic substance selected from the group comprising sodium perchlorate, sodium iodide, potassium iodide, guanidinium (iso)thiocyanate, and guanidine hydrochloride. In terms of the present invention, a significant amount of a chaotropic substance preferably is an amount of 1 M or more, more preferably of 0.5 M or more, even more preferably of 0.1 M or more, and most preferably of 0.05 M or more. Preferably, neither the binding mediating agent nor any other compound or component added to the sample during the procedure comprises any chaotropic substance. It is further preferred, that the original sample, e.g. the agarose gel band as excised from the gel prior to any further processing, does not comprise more than 1 M, preferably not more than 0.5 M, more preferably not more than 0.1 M, and even more preferably not more than 0.05 M of any of the aforementioned chaotropes. Most preferably the agarose gel matrix does not comprise any chaotropes at all.

After at least partly liquefying the agarose matrix according to the present invention, the sample may be contacted with the solid phase and optionally may be incubated in contact with said solid phase. The solid phase comprising the bound nucleic acid may then be separated essentially from any remaining parts of the sample not bound to the solid phase, such as for example remaining agarose fragments, salts, proteins, etc. To complete separation of the nucleic acid bound to the solid phase from any remaining contaminants the solid phase optionally may be washed. After washing, it may optionally be dried, before the nucleic acid preferably may be eluted from the solid phase. Thus, the method of the present invention may preferably comprise the following steps:
a) at least partly liquefying the agarose matrix,
b) contacting the sample with a solid phase, which binds the nucleic acid in presence of a binding mediating agent,
c) optionally incubating the sample in contact with the solid phase,
d) separating the solid phase essentially from any remaining parts of the sample not bound to the solid phase,
e) optionally washing the solid phase,
f) optionally drying the solid phase, and
g) eluting the nucleic acid from the solid phase.

It may be preferred to essentially remove the at least partly liquefied agarose matrix or the agarose fragments obtained in the process of partly liquefying the agarose matrix before contacting the sample with a solid phase. The term "essentially removing" indicates that at least 80%, preferably at least 85%, more preferably at least 90% and most preferably at least 95 % of the agarose matrix are removed, based on the amount of agarose present in the sample prior to step a) of liquefying the sample. The step of removing these fragments may be carried out by any method known to a person skilled in the art for this purpose.

Preferably, the agarose fragments / the solubilized and/or melted agarose may be precipitated from the sample. Thus, the method of the present invention may comprise the further steps of:
a ii) mixing the sample with an agarose flocculant/precipitating agent to flocculate/precipitate the liquefied agarose from the sample, and
a iii) optionally removing the flocculated/precipitated agarose from the sample,
wherein the additional steps are preferably carried out after liquefying the agarose matrix, but before contacting the sample with a solid phase according to step b) of the method. For removing the precipitate/flocculate any method known to a person skilled in the art for separating solid from liquid compounds may be used,
such as for example centrifugation, filtration, decantation etc., without being limited to these.

The binding mediating agent may be mixed with the sample before or during any of the steps of liquefying the agarose matrix, mixing the sample with a flocculant/precipitating agent, removing the precipitated agarose from the sample and/or contacting the sample with the solid phase according to any of steps a), a ii), a iii) or b). If the agarose matrix is liquefied by melting, the binding mediating agent may be added to the sample before melting the agarose matrix.

The binding mediating agent may preferably comprise at least one organic cation, preferably from the group comprising cationic polyamines and cationic surfactants. Preferably cationic polyamines obtained by protonation of polyamines of the general formula R¹R²N[(CH₂)₂₋₆NR³]ₙ(CH₂)₂₋₆NR⁴R⁵ may be applied, wherein R¹, R², R³, R⁴ and R⁵ may be the same or different, and independently represent hydrogen or a C₁-C₂₀ alkyl group, preferably hydrogen or a C₂-C₁₀ alkyl group, more preferably hydrogen or a C₃-C₆ alkyl group, and n represents an integer ranging from 0 to 6, which means n = 0, 1, 2, 3, 4, 5 or 6. If n > 1, the polyamines comprise more than one subunit of the formula [(CH₂)₂₋₆NR³], and R³ may be the same or different in each of these subunits. Preferably polyamines are applied which are cationic at any pH in the range of from pH 1 to pH 14, such as oligomeric ethylenimines, for example spermine, spermidine, norspermidine and putrescine.

Preferred cationic surfactants may comprise the general structure R¹R²R³R⁴N⁺X⁻, wherein R¹, R², R³, and R⁴ independently represent a C₁-C₂₀ alkyl group and X⁻ represents a negatively charged counter ion. The negatively charged counter ion can be organic or inorganic, and preferably may be an inorganic counter ion, more preferably selected from the group comprising Cl⁻ and Br⁻, most preferably representing Br⁻.

Preferably R¹ may represent a linear C₈-C₁₆-alkyl chain, in particular n-octyl, n-nonyl, n-decyl, n-undecyl, n-dodecyl, n-tridecyl, n-tetradecyl, n-pentadecyl, and n-hexadecyl and R², R³, and R⁴ independently may represent methyl (Me) or ethyl (Et). Preferably the cationic surfactant may be selected from the group comprising octyltrimethylammoniumbromide (OTAB), dodecyltrimethylammonium bromide (DTAB), tetradecyltrimethylammonium bromide (TTAB), and cetyltrimethylammonium bromide (CTAB) or a mixture thereof, and most preferably may represent CTAB.

If in the present application a range of numbers is shown, said range means that each of the members within the range shall be considered as individually described. E.g. a range of C₁-C₂₀ means C₁, C₂, C₃, C₄, C₅, C₆, C₇, C₈, C₉, C₁₀, C₁₁, C₁₂, C₁₃, C₁₄, C₁₅, C₁₆, C₁₇, C₁₈, C₁₉, C₂₀, a range of C₁-C₁₀ means C₁, C₂, C₃, C₄, C₅, C₆, C₇, C₈, C₉, C₁₀, a range of C₈-C₁₆ means C₈, C₉, C₁₀, C₁₁, C₁₂, C₁₃, C₁₄, C₁₅, C₁₆, and a range of C₃-C₆ means C₃, C₄, C₅, C₆.

As already mentioned, the step of at least partly liquefying the agarose matrix in the sample according to step a) may be carried out under heat supply, preferably by melting the agarose matrix, more preferably at a temperature in the range of from 30 to 95 °C, even more preferably in the range of from about 60 to about 80 °C. Preferably, the whole agarose matrix may be liquefied. The temperature to be applied for melting the agarose matrix depends on the kind and amount of agarose used for preparing the matrix, which is well known to a person skilled in the art. The method of the present invention is applicable to both, low-melting-temperature (LMT) agarose, comprising hydroxyethyl groups in the polysaccharide chain, thus melting at around 65 °C or even below, as well as to other agarose matrices not comprising a plurality of such groups and thus melting at considerably high temperatures. In particular if nucleic acids are to be isolated which are prone to degradation, LMT agarose may be preferred.

The agarose flocculants/precipitating agent may preferably be selected from the group comprising sodium citrate, including the mono-, di- and trisodium salt, sodium sulfite and water-soluble cosmotropic salts, preferably water-soluble cosmotropic salts comprising sulphates, nitrates, and phosphates or a mixture thereof, and most preferably may represent trisodium citrate, sodium sulfite, ammonium sulphate, sodium sulphate or lithium sulfate. Cosmotropic (also called anti-chaotropic) substances stabilize proteins, decrease the solubility of non-polar substances in water and enhance hydrophobic interactions, just contrary to a chaotropic substance. Accordingly, cosmotropic ions are found on the left hand side of the Hofmeister series. The agarose flocculant/precipitating agent may for example be added to the sample comprising the at least partly liquefied agarose in form of an aqueous solution. The concentration of the flocculant/precipitating agent in said solution to be added to the sample preferably is above 1.2 M, and more preferably may be in the range of between 1.5 M and 10 M, even more preferably in the range of from 2 M to 4 M. Preferably about 2 to 5 volumes of such an aqueous agarose flocculant/precipitating agent is mixed with the at least partly liquefied sample. As 100 mg of an agarose gel equal approximately 100 µL, the volume of the precipitating solution to be added can be easily determined using a scale. After mixing the agarose flocculant/precipitating agent with the sample and optionally incubating said mixture to complete precipitation, preferably at room temperature (23 ± 2 °C) or temperatures below room temperature, the precipitate may then be removed from the sample as described above.

The binding mediating agent may be provided in form of an aqueous solution. Preferably said aqueous solution comprises from about 0.001 to about 5 wt.-% of one or more binding mediating agent(s) mentioned above, preferably from about 0.005 to about 1 wt.-%, more preferably from about 0.01 % to about 0.6 % and most preferably from about 0.01 % to about 0.4 %. Using CTAB, the concentration may for instance even be as low as 0.02 % or less, e.g. from about 0.01 to 0.02 wt.-%. In contrast to chaotrope mediated binding known from the state of the art, in the present invention rather small concentrations of the binding mediating agent in the sample are sufficient for selectively and (almost) quantitatively binding the nucleic acids to the solid phase. Typically, a concentration of less than 1 M, preferably less than 0.1 M and even more preferably less than 0.01 M of the binding mediating agent in the sample may be used. For example one volume of a binding mediating agent solution comprising 0.01 wt.-% CTAB (corresponding to 5.5 mM/L) are sufficient to selectively and essentially quantitatively bind nucleic acid fragments of all different sizes from a standard agarose gel to a nucleic acids binding solid phase. Preferably said aqueous binding mediating agent solution may comprise less than 2 M of any inorganic salt, more preferably less than 0.5 M, even more preferably less than 0.1 M, and most preferably said binding mediating agent solution is free of any inorganic salt. Preferably said binding mediating agent solution may comprise less than 20 % of any alcohol, more preferably less than 5 %, even more preferably less than 1 % and most preferably said binding mediating agent solution does not comprise any alcohol at all. Said aqueous binding mediating agent may comprise stabilizers, such as for example sodium azide.

The solid phase may be selected from the group comprising beads, including magnetic beads, particulates, powders, layers, meshes, tips, dipsticks, tubes, container walls, filters, gels, ion exchange resins, reversed phase resins, matrices, and membranes, preferably from the group comprising silica beads, magnetic beads coated with a silica layer, silica beads further functionalized with negatively charged polymers, silica slides, silica powder, quartz filter paper, quartz wool, silica membranes, and silica matrices, and most preferably may represent a silica membrane or a silica matrix. Preferably, said silica membrane or silica matrix may be comprised in a column or filtration device, more preferably in a spin column. These most preferred embodiments allow a fast and automatic handling of a plurality of samples in high throughput processing. In principle, any solid phase suitable for binding nucleic acids in the presence of a high concentration of chaotropic substance may be used in the method for the present invention, a plurality of which is well known to a person skilled in the art.

After binding the nucleic acids to the solid phase, at least one, preferably two or more washing steps may be carried out to remove contaminants from the nucleic acids bound to the solid phase. Preferably these washing steps may be carried out by using washing liquid(s)/washing buffer(s) selected from the group comprising water, aqueous solutions comprising an alcohol, preferably up to 30%, preferably 0.01 to 25% (v/v) of a C₁-C₅-alcohol including methanol, ethanol and isopropanol, a buffering agent, a chelating agent or mixtures thereof, e. g. acidic & basic buffering solutions such as for example commercially available TRIS, MES, MOPS or HEPES buffer, without being limited to these.

For example, after binding the nucleic acids to the solid phase, the solid phase may first be washed using nuclease-free water or the commercially available buffer PE (Qiagen, Hilden, Germany), followed by a second washing step using an aqueous solution comprising a C₁-C₅-alcohol, including methanol, ethanol and isopropanol in a concentration of ≥ 80% (v/v). The step of washing with a concentrated alcohol solution removes the binding mediating agent from the solid phase while simultaneously precipitating the nucleic acids on the solid phase. Accordingly, it is preferred to quickly carry out this particular washing step, i.e. in about 15 sec to 1.5 min, preferably at a high centrifugal force of about 10,000 xg or above, if a spin column comprising the solid phase is used for separating the nucleic acid from the sample.

After CTAB mediated binding the nucleic acids may be eluted from the solid phase using aqueous solutions of ethanol, comprising 40 wt.-% or more of ethanol. The unintended elution of nucleic acids bound to the solid phase during ethanol washes for removing CTAB can be prevented by a fixative wash, using an aqueous solution comprising about 15 to 25 % of a C₁-C₅ alcohol, including methanol, ethanol, n-propanol, iso-propanol, n-butanol, and n-pentanol, preferably ethanol and about 3 to 10 mM, preferably about 5 to 7 mM spermine or spermidine. After this fixative wash, the nucleic acid may be eluted using pure water, if the above mentioned cationic surfactants had been used as fixatives, or a low salt buffer (comprising of from about 45-to about 90 mM/L sodium chloride for example) for all of the above-mentioned fixatives. In an acidic environment (pH < 7) binding of the nucleic acids to the solid phase is stronger than in an alkaline environment (pH > 7), and a higher amount of eluting agent (and/or a higher concentration of the eluting substance(s) therein) may be necessary.

If a washing step using an aqueous alcoholic solution has been employed, it may be preferred to optionally dry the solid phase, for example by incubating the solid phase at room temperature, dry centrifugation, incubating at elevated temperature and/or reduced pressure and so on, before eluting the nucleic acids from the solid phase.

A further advantage of the method of the present invention is the fact that the step of eluting the nucleic acids from the solid phase may be carried using a low salt eluting buffer or even water, in particular if the above mentioned cationic surfactants had been used as fixatives. Thus, the step of eluting the nucleic acids from the solid phase according to step g) may be carried out using an aqueous solution comprising less than 1 M of salt, said solution preferably comprising one or more buffering agents, e.g. selected from the group comprising Tris-HCl, MOPS, MES and HEPES, and optionally a chelating agent, preferably selected from the group comprising EDTA, EGTA, citrate and so on, without being limited to these. A typical elution buffer may for example comprise about 10 mM of a buffering substance and about 1 mM of a chelating agent, adjusted to pH 7.5-9. In principle, all low salt eluting buffers known from the state of the art may be used in the present invention, provided that they do not comprise any of the chaotropic substances mentioned above.

Using the method of the present invention RNA, DNA, as well as engineered nucleic acids such as protein-nucleic acids (PNA) and locked nucleic acids (LNA) can be extracted from the respective agarose samples. Preferably the nucleic acids may be DNA, including eukaryotic, prokaryotic and viral DNA, preferably selected from the group comprising genomic DNA, amplified DNA, expressed DNA, bio-engineered DNA, episomal DNA, plasmid DNA, mitochondrial DNA, cDNA, or a fragment thereof or a mixture thereof.

The invention further provides a kit for extracting nucleic acid(s) from a sample comprising gelled agarose and one or more nucleic acid(s), preferably from agarose gels, comprising: a) a chaotrop-free binding mediating agent, preferably a binding mediating agent as already described above and, b) a solid phase capable of binding nucleic acids in the presence of the binding agent, preferably a solid phase as already described above.

In addition, the kit further may comprise c) at least one agarose flocculant/precipitating agent, preferably an agarose flocculant/precipitating agent as already described above, and/or d) one or more washing liquid(s)/washing buffer(s), preferably selected from the group as already mentioned above, and/or e) at least one elution buffer, preferably an elution buffer as already mentioned above, and/or f) instructions for using the kit.

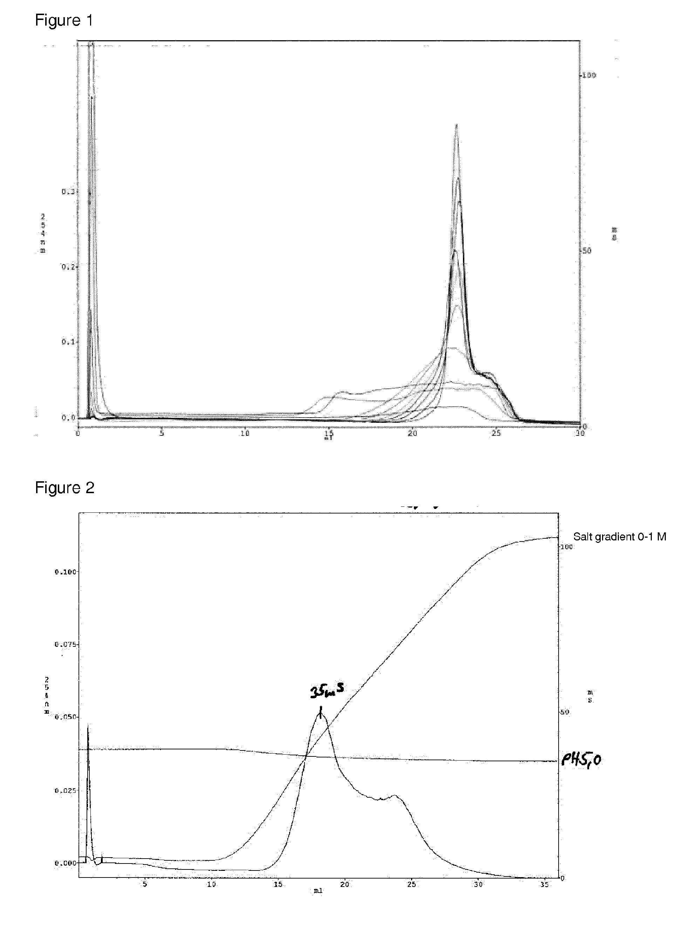

Figure 1 shows elution profiles of DNA bound to a GF/D membrane, pretreated with different concentrations of CTAB. The effect of the CTAB concentration on the binding of DNA to a GF/D membrane commercially available from GE Healthcare (Chalfont St. Giles, UK) increases the binding capacity (see Example 1).

Figure 2 shows an elution profile illustrating the binding of RNA to a GF/D membrane commercially available from GE Healthcare (Chalfont St. Giles, UK) in the presence of CTAB using a salt gradient for elution (see Example 1).

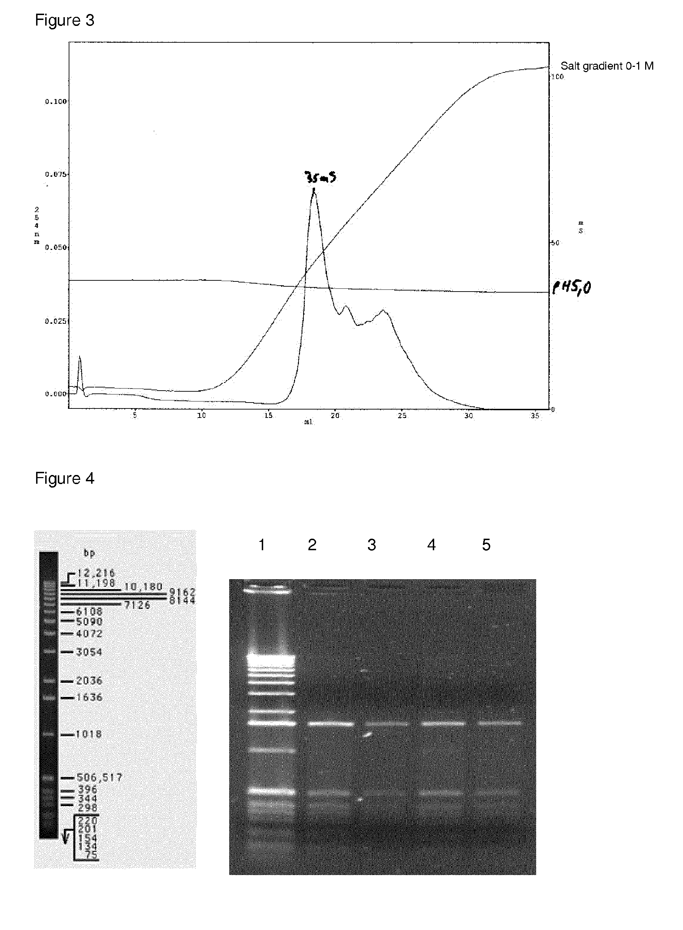

Figure 3 shows an elution profile illustrating the binding of RNA to a GF-51 membrane commercially available from GE Healthcare (Chalfont St. Giles, UK) in the presence of CTAB using a salt gradient for elution (see Example 1).

Figure 4 shows the elution of a 1 kb DNA size marker (a guide to the size ladder is given on the left) from a commercially available spin column (QIAquick, QIAGEN, Hilden, Germany) comprising a silica based solid phase after CTAB-mediated binding and fixative washing using 6 mM spermidine and 20 % aqueous ethanol (v/v) (see Example 2).

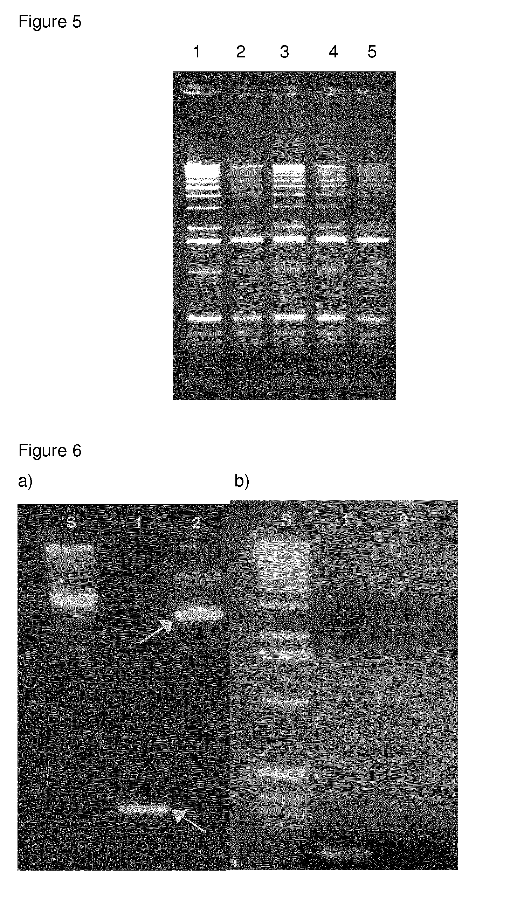

Figure 5 shows the elution of a 1 kb DNA size marker from a commercially available column (QIAquick, QIAGEN, Hilden, Germany) using the method of the present invention without fixative spermidine washes (see Example 3).

Figure 6 a) shows an electrophoresis gel of a commercially available size marker (lane S), a 100 bp PCR fragment (lane 1) and the PBR322 vector (lane 2). Figure 6 b) shows the electrophoresis gel of the nucleic acids isolated using the method of the present invention from the marked bands of the gel shown in Fig. 6 a) (see Example 4).

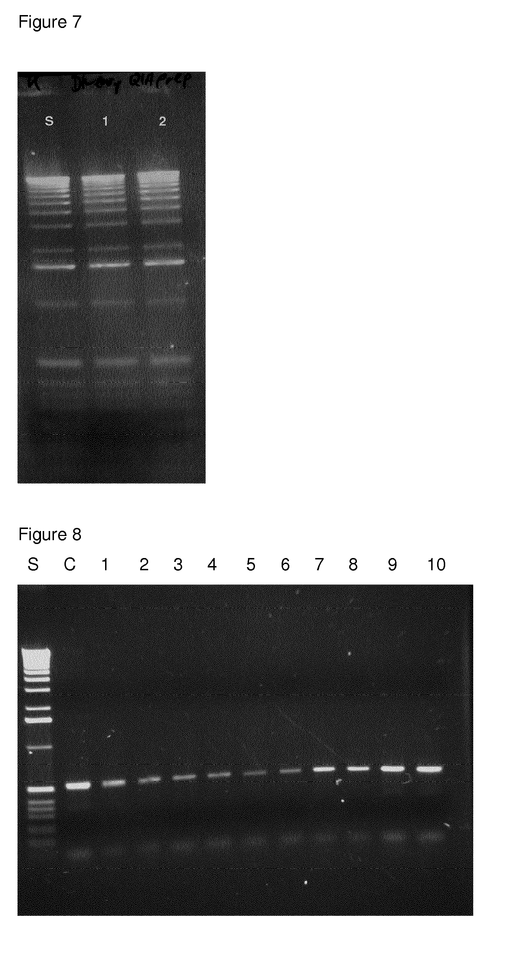

Figure 7 shows the electrophoresis gel of samples obtained by isolating a commercially available size marker (lane S) from an Agarose gel using the method of the present invention in combination with two commercially available spin columns (lane 1: DNeasy, lane 2: QIAquick, both from QIAGEN, Hilden, Germany; see Example 5).

Figure 8 shows an electrophoresis gel of a commercially available size marker (lane S), a control (lane C) and a 500 bp DNA fragment isolated from agarose gel bands using different liquefying protocols according to the present invention and the state of the art (lanes 1 and 2: centrifugation protocol, lanes 3 and 4 melting protocol, lanes 5 and 6: alkaline melting protocol, lanes 7 and 8: QIAquick protocol and lanes 9 and 10: QIAex protocol) (see example 6)

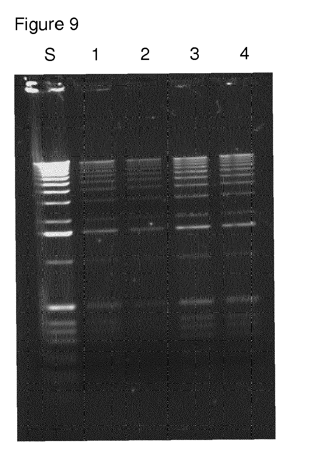

Figure 9 shows the result of isolating a DNA size ladder from an agarose matrix using either CTAB (lanes 1 and 2) or OTAB (lanes 3 and 4) in the method of the present invention (example 7). In lane S, pure size marker was applied for the sake of comparison.

### Examples

### Example 1: Effect of CTAB concentration on binding

A commercially available GF/D membrane (GE Healthcare, Chalfont St. Giles, UK) was cut to size using a scalpel and fixed inside an HPLC column using a filter holder made of PEG. The membrane was equilibrated with a solvent mixture comprising x % (v/v) of an aqueous solution of CTAB (0.02 wt.-%), 90 - x % (v/v) of water and 10% (v/v) of tenfold concentrated buffer TAE (TAE 10x, pH 8.5) at a flow rate of 1.5 mL/min, wherein x is 0, 10, 20, 30, 40, 50, 60, 70, 80 or 90% (v/v). The column was then loaded with samples comprising 50 µg pQ51 plasmid DNA and 0.01% CTAB. Excess CTAB was removed from the column by flushing it with 4 mL of a solvent mixture comprising 90% (v/v) of water and 10% (v/v) of buffer TAE 10X at the above mentioned flow rate. The nucleic acid were then eluted from the column at the above mentioned flow rate using 20 mL of a solvent mixture comprising water and 10% (v/v) of buffer TAE 10 x and sodium chloride in an amount linearly increasing from 0 to 1 M during elution (13.3 min).

The chromatographic traces recorded at a wavelength of 254 nm are presented in figure 1 in an overlaid fashion. With increasing amounts of CTAB the capacity and efficiency of the DNA binding to GF/D membrane increases, as can be seen by the sharpness of the peak at about 22 - 23 mL elution volume which increases with an increasing amount of CTAB in the elution buffer.

RNA can be bound to silica membranes in the presence of CTAB as well: 25 mg RNA from E. *coli* were loaded to the commercially available GF/D and GF-51 membranes (both, GE Healthcare, Chalfont St. Giles, UK) at pH 5 in an 50 mM potassium acetate buffer additionally comprising 0.01 wt% CTAB. The RNA was eluted from the column using a gradient of sodium chloride (increasing the concentration of sodium chloride in the buffer from 0 M to 1 M during the elution time of 13.3 min) in the above mentioned acetate buffer. For both columns, elution maximum was seen at a conductivity of 35 mS, as shown in figure 2 for the GF/D membrane and figure 3 for the GF-51 membrane.

### Example 2: Purification of DNA fragments of small size using a fixative wash

100 µL of liquid 0.8 % agarose in TAE buffer was mixed with 1 µL of the commercially available 1 kb-ladder (Invitrogen, Darmstadt, Germany) and 100 µL of an aqueous solution of CTAB (comprising 0.02 wt.-% CTAB). The agarose was precipitated by adding 300 µL of an aqueous 3 M ammonium sulphate solution. The precipitate may be separated, for example, by centrifugation. This, however, is not necessary. In the present example, the whole mixture was added to a commercially available QIAquick column (Qiagen, Hilden, Gemany) and the column was centrifuged for 3 min at 700 xg. The column was then washed with 300 µL of an 20 % aqueous ethanol solution further comprising 6 mM spermidine for 3 min at 700 xg. Then the column was washed with 300 µL of the commercially available buffer PE (Qiagen, Hilden, Germany) at 18.620 xg to remove the CTAB for 1 min. Thereafter the column was dried by centrifuging at the same speed for 3 min. After drying, the DNA was eluted using 200 µL of the commercially available TE buffer (Qiagen, Hilden, Germany).

The eluates obtained from four runs were evaluated on an agarose gel, presented in figure 4 (lanes 2 to 5). In lane 1, an aliquot of the 1 kb ladder used for isolation was directly applied to the gel as a size marker. It can be seen, that after a fixative wash DNA fragments smaller than 1.6 kb selectively can be eluted from the column, while larger fragments remain bound to the silica matrix, since no DNA was detected in the flow through during all binding and washing steps as evaluated by gel electrophoresis of the eluates (data not shown).

### Example 3: Elution without a previous fixative wash

Four samples were prepared and loaded onto the columns as described in example 2, but instead of the fixative spermidine/ethanol wash the column was washed using 400 µL of water or 20 % aqueous ethanol solution, respectively (2 samples each) at 2.500 xg. The PE wash and drying was performed as described in example 2. Thereafter DNA fragments of all sizes were eluted using 100 µL of a salt-free TE buffer. The eluates were analyzed on an agarose gel shown in figure 5. In lane 1 the 1 kb ladder was directly applied to the gel as a size marker. The samples as analyzed were obtained after a spermidine-free water wash (lanes 2 and 3), or a washing step using a 20% aqueous ethanol solution free of spermidine (lanes 4 and 5). It can be seen that fragments of any size can be easily eluted if no fixative wash is applied. Conductivity measurements showed that also these non-fixative washing steps are efficient in removing ammonium sulphate from the samples (data not shown).

### Example 4: Isolation of short DNA fragments and plasmid DNA from an agarose gel

On an 1% agarose gel (low-melting agarose from Sigma-Aldrich, St. Louis, MO, USA), comprising 2.5 µL gel red per 50 mL agarose gel, 200 ng of samples of a 100 bp PCR fragment (lane 1 in figure 6a) and the PBR322 vector (lane 2 in figure 6a) were run. In lane S an 1 kb ladder (Invitrogen, Darmstadt, Germany) was run as a size marker. The bands marked with an arrow in Fig. 6a were excised from the gel and to each gel fragment an equivalent volume of an 0.04 wt.-% aqueous solution of CTAB (200 µL) was added. The obtained mixture were incubated at 65 °C for 10 min and mixed by vortexing to solubilize the agarose. 400 µL of an aqueous solution of ammonium sulphate (3 M) was added to precipitate the agarose. 400 µL of the 0.04 wt-.% aqueous solution of CTAB was then added to obtain a final concentration of about 0.02% (w/v) CTAB.

Commercially available QIAprep columns (Qiagen, Hilden, Germany) were equilibrated by passing 200 µL of the 0.04 wt-.% aqueous solution of CTAB through it by spinning the column at 6,000 xg for 1 min.

Each of the above samples was loaded onto such a column, and the columns were spun at 6,000 xg for 1 min. The flow-through was discarded. To remove residual ammonium sulphate, 500 µL of water was loaded onto the columns and the columns were spun at 6,000 xg for 1 min. To remove CTAB 500 µL of commercially available buffer AW2 (Qiagen, Hilden, Germany) was loaded onto the columns and the columns were spun at 20,000 xg for 3 min. Elution of DNA was carried out using 50 µL of commercially available buffer TE (Qiagen, Hilden, Germany), spinning the columns at 6,000 xg for 1 min, re-loading the obtained eluates onto the columns and spinning them at 6,000 xg for 1 min again. The eluates were collected and 10 µL from each sample was loaded onto an agarose gel prepared as described above, which is shown in figure 6b. It can be seen that both, the 100 bp DNA fragment as well as the plasmid, can be recovered from an agarose gel using the method of the present invention.

### Example 5: Isolation of a 1kb DNA ladder from an agarose gel

Agarose gel samples were prepared by melting 9 mg low melting agarose (Sigma-Aldrich) in the presence of 90 µL 1 x TAE buffer. To each sample 1 µL (1 µg) of a 1 kb DNA ladder (Invitrogen) was added and the samples were then cooled on ice to solidify the agarose.

To each sample 90 µL 0.04 % CTAB (aq) was added and the samples were incubated at 65 °C to melt the agarose gel. 180 µL of an aqueous solution of ammonium sulphate (3 M) was added, followed by 180 µL of the 0.04 wt-.% aqueous solution of CTAB. Each sample was spun at 3,000 xg for 10 seconds to pellet the agarose.

Both, a QIAprep as well as a DNeasy column (both commercially available from Qiagen, Hilden Germany) were equilibrated by passing 100 µL of the 0.04 wt-.% aqueous solution of CTAB through them by spinning at 6,000 xg for 1 min.

The above samples were each loaded onto the columns, and the columns were spun at 6,000 xg for 1 min. The flow-through was discarded. 500 µL of water was loaded onto the columns and the columns were spun at 6,000 xg for 1 min. 500 µL of commercially available buffer AW2 (Qiagen, Hilden, Germany) was then loaded onto the columns and the columns were spun at 6,000 xg for 3 min. The columns were dried by further spinning them at 20,000 xg for 1 min. Elution of DNA was carried out using 100 µL of commercially available buffer TE (Qiagen, Hilden, Germany), spinning the columns at 6,000 xg for 1 min, re-loading the obtained eluates onto the columns and spinning them at 6,000 xg for 1 min again. The eluate was collected and 15 µL of the eluate obtained from each sample was loaded onto an agarose gel prepared as described above, which is shown in figure 7. In lane 1 the results obtained using the DNeasy column and in lane 2 the results obtained using the QIAprep column are shown. In lane S a control obtained by diluting 1 µL of the 1 kb ladder in 100 µL buffer TE and directly applying 15 µL of this solution to the gel is shown.

### Example 6: Variation of liquefying conditions

Excised agarose bands comprising a 500 bp DNA fragment were liquefied according to the procedures described below:
*1. Centrifugation protocol:* One volume of a 0.04 wt.-% aqueous solution of CTAB was added to the sample. The mixture was placed into a QIAprep column and spun at full speed for 3 min. The column was washed two times using 500 µL of buffer AW2 (Qiagen, Hilden, Germany), removing the washing buffer by spinning at 7,000 xg for 1 min. The column was dried by spinning it at full speed for 3 min. Then the DNA was eluted using 100 µL of buffer TE (Qiagen, Hilden, Germany).
*2. Melting protocol:* One volume (based on the volume of the sample to be melted) of a 0.04 wt.-% aqueous solution of CTAB was applied to the sample, and the mixture was incubated for 10 min at 95 °C. Two volumes (based on the original volume of the sample to be melted) of an aqueous solution of ammonium sulphate (3 M) were added to precipitate the dissolved agarose, and one volume (based on the original volume of the sample to be melted) of the 0.04 wt.-% aqueous solution of CTAB was added. The mixture was transferred to a QIAprep column and the sample was further processed as described in the centrifugation protocol.
*3. Alkaline melting protocol:* This protocol is essentially the same as described under item 2 for the (simple) melting protocol, except that 20 mg TRIS were added for each 150 mg of sample. TRIS may be added separately, e.g. after adding the aqueous solution of CTAB to the sample, or may be added to the CTAB solution before adding said solution to the sample.

In addition, further samples were processed using the QIAquick and the QIAex protocol and equipment according to the manufacturer's instructions, respectively.

The DNA recovered from these samples was analyzed on an agarose gel which is shown in figure 8. A size marker is shown in lane S, a control is shown in lane C and the 500 bp DNA fragment isolated from agarose gel bands using the different liquefying protocols in lanes 1 to 10 (lanes 1 and 2: centrifugation protocol, lanes 3 and 4 melting protocol, lanes 5 and 6: alkaline melting protocol, lanes 7 and 8: QIAquick protocol and lanes 9 and 10: QIAex protocol). It can be seen that various different methods for liquefying the agarose matrix can be employed in the method of the present invention, all of them capable of providing DNA of very good integrity and yield. For instance, heating may be applied for liquefying, but is not necessary. As well, flocculating or precipitating of the liquefied agarose may be carried but neither is mandatory.

### Example 7: Varying the kind of binding mediating agent

A commercially available DNA size marker (1 kb DNA, Gibco, Invitrogen) was extracted from a sample comprising gelled agarose using aqueous solutions of either CTAB or OTAB at a concentration of 0.4 % (w/v), respectively, using the centrifugation protocol described in example 6, except that 300 µL of buffer AW2 were used in the washing step and 150 µL of buffer TE were used for eluting the DNA.

The obtained eluates were analyzed on an agarose gel shown in figure 9. The results obtained using CTAB are shown in lanes 1 and 2, those obtained using OTAB in lanes 3 and 4. In lane S, pure size marker was applied for the sake of comparison. This example demonstrates that using OTAB very good results may be obtained as well.

It can be seen that using the method of the present invention DNA fragments of a large size span can be recovered from agarose gels in a fast and efficient manner. Furthermore, the method of the present invention may as well be carried out using solid phases already commercially available, such as for example spin columns comprising silica membranes.

In the method of the present invention, neither chaotropic salts nor further toxic chemicals such as phenol, chloroform etc. are needed. As the compounds to be applied according to the present invention are readily available and cheap or only have to be used in small amounts, the method of the present invention also is attractive from an economic point of view. In addition, a highly purified nucleic acids eluate is obtained comprising a only very small amount of salts and thus can be used without further processing in a plurality of various methods for detecting, analysing and/or amplifying nucleic acids.

## Claims

1. A method for extracting one or more nucleic acids from a sample comprising gelled agarose (agarose matrix) and at least one nucleic acid, preferably from agarose gels, comprising the steps of:
- at least partly liquefying the agarose matrix, and
- separating the nucleic acid from the sample using a solid phase, which selectively binds the nucleic acid in presence of a binding mediating agent,
**characterized in that** neither the binding mediating agent nor any other compound added to the sample during the procedure comprises a chaotropic substance, selected from the group consisting of sodium perchlorate, sodium iodide, potassium iodide, guanidinium thiocyanate, and guanidine hydrochloride.

2. The method according to claim 1, comprising the steps of
a) at least partly liquefying the agarose matrix,
b) contacting the sample with a solid phase, which binds the nucleic acid in presence of a binding mediating agent,
c) optionally incubating the sample in contact with the solid phase,
d) separating the solid phase essentially from any remaining parts of the sample not bound to the solid phase,
e) optionally washing the solid phase,
f) optionally drying the solid phase, and
g) eluting the nucleic acid from the solid phase.

3. The method according to claim 1 or 2, further comprising the steps of:
a ii) mixing the sample with an agarose flocculant/precipitating agent to flocculate/precipitate the liquefied agarose from the sample, and
a iii) optionally removing the flocculated/precipitated agarose from the sample.

4. A method according to any of claims 1 to 3, further comprising the step of mixing the sample with said binding mediating agent before or during any of the steps of liquefying the agarose matrix, mixing the sample with the flocculant/precipitating agent, removing the precipitated agarose from the sample and/or contacting the sample with the solid phase according to any of steps a), a ii), a iii), or b).

5. The method according to any of claims 1 to 4, wherein the binding mediating agent comprises at least one organic cation, preferably selected from the group comprising cationic polyamines and cationic surfactants, more preferably selected from the group comprising spermine, spermidine, norspermidine, putrescine, and cationic surfactants of the general structure R¹R²R³R⁴N⁺X⁻, wherein R¹, R², R³, and R⁴ independently represent a C₁-C₂₀ alkyl group and X⁻ represents a negatively charged counter ion.

6. A method according to claim 5, wherein the organic cation is a cationic surfactant of the formula R¹R²R³R⁴N⁺X⁻, wherein R¹ represents a linear C₈-C₁₆-alkyl chain and R², R³, and R⁴ independently represent Me or Et, preferably selected from the group comprising octyltrimethylammonium bromide (OTAB), dodecyltrimethylammonium bromide (DTAB), tetradecyltrimethylammonium bromide (TTAB), and cetyltrimethylammonium bromide (CTAB), or a mixture thereof.

7. A method according to any of claims 1 to 6, wherein the step of at least partly liquefying the agarose matrix in the sample according to step a) is carried out under heat supply, preferably by melting the agarose matrix, more preferably at a temperature in the range of from 30 to 95 °C.

8. A method according to any of claims 3 to 7, wherein the agarose flocculant/precipitating agent is selected from the group comprising sodium citrate, sodium sulfite and water-soluble cosmotropic salts, preferably water-soluble cosmotropic salts comprising sulphates, nitrates, and phosphates or a mixture thereof, and most preferably represents trisodium citrate, sodium sulfite, ammonium sulphate or sodium sulphate.

9. A method according to any of claims 1 to 8, wherein the solid phase is selected from the group comprising beads, including magnetic beads, particulates, powders, layers, meshes, tips, dipsticks, tubes, container walls, filters, gels, ion exchange resins, reversed phase resins, matrices, and membranes, preferably from the group comprising silica beads, magnetic beads coated with a silica layer, silica beads further functionalized with negatively charged polymers, silica slides, silica powder, quartz filter paper, quartz wool, silica membranes, and silica matrices, and most preferably represents a silica membrane or a silica matrix.

10. A method according to any of claims 2 to 9, wherein at least one, preferably at least two steps of washing according to step e) are carried out, preferably by using washing liquid(s)/washing buffer(s) selected from the group comprising water, aqueous solutions comprising 0.01 to 25 % of a C₁-C₅-alcohol, a buffering agent, a chelating agent or mixtures thereof.

11. A method according to any of claims 2 to 10, wherein the step of eluting the nucleic acids from the solid phase according to step g) is carried out using an aqueous solution comprising less than 1 M of salt, said solution preferably comprising one or more buffering agents, selected from the group comprising TRIS, MOPS, MES and HEPES, and optionally a chelating agent, preferably selected from the group comprising EDTA, EGTA and citrate.

12. A method according to any of claims 1 to 11, wherein the nucleic acid is DNA, including eukaryotic, prokaryotic and viral DNA, preferably selected from the group comprising genomic DNA, amplified DNA, expressed DNA, bio-engineered DNA, episomal DNA, plasmid DNA, mitochondrial DNA, cDNA, or a fragment thereof or a mixture thereof.

13. A kit for extracting nucleic acid(s) from a sample comprising gelled agarose and one or more nucleic acid(s), preferably from agarose gels, comprising:
a) a chaotrop-free binding mediating agent or a solution comprising such an agent, preferably a binding mediating agent according to claims 5 or 6, and
b) a solid phase capable of binding nucleic acids in the presence of the binding agent, preferably a solid phase according to claim 9.

14. A kit according to claim 13, further comprising:
c) at least one agarose flocculant/precipitating agent, preferably an agarose flocculant/precipitating agent according to claim 8, and/or
d) one or more washing liquid(s)/washing buffer(s), preferably selected from the group mentioned in claim 10, and/or
e) at least one elution buffer, preferably an elution buffer according to claim 11, and/or
f) instructions for using the components of the kit in a method according to any of claims 1 to 12.
